# EUROPEAN PATENT APPLICATION

(11) **EP 4 311 580 A1**
(43) Date of publication of application: **31.01.2024**
(21) Application number: 22187767.3
(22) Date of filing: 29.07.2022
(51) Int. Cl.: A61Q 11/00, A61K 8/44, A61K 8/21, A61P 1/02, A61K 33/16

(54) **AMINOACID-FLUORIDE SALTS FOR MOUTH HYGIENE**

(71) Applicant: Schäfer, Rolf, 4422 Arisdorf (CH)
(72) Inventor: SCHÄFER, Rolf, 4422 Baselland Arisdorf (CH); BLÜMEL, Lisa Anna Ruth, 18057 Rostock (DE)
(74) Representative: Braunpat AG

(57) **Abstract**

The present invention relates to novel fluoride salts of different amino acids or fluoride salts of amino acid derivatives. These fluoride salts can be used as ingredients in products for mouth hygiene and dental care, such as toothpastes, mouthwashes, and dental bleaching products. The invention further relates to such products for mouth hygiene and dental care comprising the novel fluoride salts. The invention also relates to the use of the novel fluoride salts in methods for the prevention and treatment of caries.

## Description

### FIELD OF THE INVENTION

The present invention relates to novel fluoride salts of different amino acids or fluoride salts of amino acid derivatives. These fluoride salts can be used as ingredients in products for mouth hygiene and dental care, such as toothpastes, mouthwashes, and dental bleaching products. The invention further relates to such products for mouth hygiene and dental care comprising the novel fluoride salts. The invention also relates to the use of the novel fluoride salts in methods for the prevention and treatment of caries.

### BACKGROUND OF THE INVENTION AND TECHNICAL PROBLEMS UNDERLYING THE PRESENT INVENTION

Fluorides are known to slow down caries and are therefore added to drinking water, toothpastes or nutrient salts. Enamel contains certain hydroxyapatite moieties which are attacked by acids present in certain foods or acids from sugar which are formed by mouth microorganisms. These acid sensitive moieties are exchanged by fluorides at higher concentrations (up to 1400ppm) and are therefore less acid-sensitive being more resistant to caries. As a result, in the 1940s experimental proof of concept of fluorides in water led to fluorination of drinking water in the USA and other countries. Therefore, fluoride salts were added to toothpastes, gels and liquids for mouth hygiene and decreased caries formation. Many dental hygiene products contain fluoride salts of long chain organic amines, which are very toxic on living cells already at very low concentrations in the nanomolar range (Mechanisms of action of ionic liquids on living cells: the state of the art, Pallavi Kumari, Visakh V.S. Pillai, Antonio Benedetto, Biophysical Reviews 12, 1187-1215, 2020). Taking into consideration that osteoblasts under the teeth are building up the enamel, long chain organic amines called amine fluorides (e.g. Dectaflur: 9-octadecenylamine-hydrofluoride; Olaflur: N,N,N-Tris(2-hydroxyethyl-N-octadecyl-1,3-diaminopropane-dihydrofluoride) widely used in famous brands in mouth hygiene products, may be harmful for these enamel forming cells by mechanisms of cell membrane lysis resulting in cellular death.

Thus, there was a need in the prior art for alternative fluoride salts that provide the beneficial effects of fluoride ions while at the same being less harmful to osteoblasts and other living cells.

The present invention shows for the first time that hydrofluoride salts of amino acids and amino acid derivatives are less harmful for osteoblasts than the fluoride salts of long chain organic amines used in the prior art. The present invention also describes the use of said hydrofluoride salts of amino acids and amino acid derivatives in dental hygiene products.

### SUMMARYOFTHE INVENTION

In a first aspect, the present invention is directed to a fluoride salt of an amino acid or a fluoride salt of an amino acid ester.

In a second aspect, the present invention is directed to the fluoride salt of an amino acid or the fluoride salt of an amino acid ester according to the first aspect for use in medicine.

In a third aspect, the present invention is directed to the fluoride salt of an amino acid or the fluoride salt of an amino acid ester according to the first aspect for use in the prevention or treatment of caries.

In a fourth aspect, the present invention is directed to a use of the fluoride salt of an amino acid or the fluoride salt of an amino acid ester according to the first aspect for mouth hygiene and/or dental care, wherein the use is non-therapeutic.

In a fifth aspect, the present invention is directed to a composition for mouth hygiene and/or dental care comprising the fluoride salt of an amino acid or the fluoride salt of an amino acid ester according to the first aspect, and optionally further comprising one or more of the following components:
a preservative (e.g. polyhexanide), a gelling agent (e.g. carbopol 940), a flavouring agent (e.g. *Mentha piperita* oil), a pH balancing agent (e.g. triethanolamine), a humectant (e.g. glycerol), a bleaching agent (e.g. carbamide peroxide), or a polishing agent (e.g. mica).

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### Definitions:

As used herein, the term *"amino acid"* refers to naturally occurring and synthetic amino acids, as well as amino acid analogs and amino acid mimetics, all of which are characterized by containing amine and carboxyl functional groups and a side chain (R) specific to the particular amino acid. In preferred embodiments of the present invention, the term *"amino acid"* refers to naturally occurring amino acids. In further preferred embodiments of the present invention, the term *"amino acid"* refers to any one of the 20 proteinogenous amino acids that are encoded by the genetic code (i.e. alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine).

As used herein, the term *"about"* refers to numerical values ranging from 5% below the indicated numerical value to 5% above the indicated numerical value. For example, a concentration of *"about 1400 ppm"* encompasses a concentration ranging from 1330 ppm to 1470 ppm.

Throughout this description, concentrations are sometimes presented as percentages (%). If not explicitly indicated otherwise, % concentrations are %(w/v) concentrations. This means the concentration is defined as percent weight per volume; e.g. as g per 100 mL.

As used herein, a "derivative" of an amino acid refers to a compound in which one or more functional groups of the amino acid have been reacted with non-toxic compounds. Examples of such derivatives are esters (e.g. esters of the carboxy group with alcohols, such as ethanol; or esters of the alcoholic amino acids serine or threonine with a carboxylic acid, such as acetic acid) or thioesters of the mercapto group of cysteine with a carboxylic acid, such as acetic acid.

As used herein, "an amino acid ester" refers to a compound in which the alpha carboxy group of the amino acid has been esterified with a C₁ to C₈ alcohol, preferably with a C₁ to C₆ alcohol, more preferably with a C₁ to C₄ alcohol, even more preferably with a C₁ to C₂ alcohol, and most preferably with a C₂ alcohol (i.e. with ethanol).

As used herein, "an ethyl ester of an amino acid" refers to a compound in which the alpha carboxy group of the amino acid has been esterified with ethanol.

As used herein, "treat", "treating" or "treatment" of a disease or disorder means accomplishing one or more of the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease or disorder means preventing that a disease or disorder occurs in a subject for a certain amount of time. For example, if a compound of the invention (or a pharmaceutical composition comprising the compound) is administered to a subject with the aim of preventing a disease or disorder, said disease or disorder (e.g. caries) is prevented from occurring at least on the day of administration and preferably also on one or more days (e.g. on 1 to 30 days; or on 2 to 28 days; or on 3 to 21 days; or on 4 to 14 days; or on 5 to 10 days) following the day of administration.

As used herein, the term "subject" includes any animal who/which may benefit from a treatment with the compound described herein (i.e. with a fluoride salt of an amino acid or of an amino acid ester). The term subject particularly includes any animal having teeth, especially any mammal having teeth. Preferably, a "subject" is selected from the group consisting of laboratory animals (e.g. mouse or rat), domestic animals (including e.g. guinea pig, rabbit, pig, sheep, goat, camel, cow, horse, donkey, cat, or dog), or primates including monkeys, apes and human beings. It is particularly preferred that the "subject" is a human being. The terms "patient" and "subject to be treated" (or in short: "subject") are used interchangeably herein.

### Embodiments of the Invention:

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect the present invention is directed to a fluoride salt of an amino acid or a fluoride salt of an amino acid ester.

In one embodiment of the first aspect, the fluoride salt of an amino acid is selected from the group consisting of: Histidine-ammonium-difluoride, Tryptophan-N-ammonium-mono-hydrofluoride, Lysine-ε-ammonium fluoride; decalysine-ε-ammonium-decafluoride.

In one embodiment of the first aspect, the fluoride salt of an amino acid ester is a fluoride salt of an ethyl ester of an amino acid. In a further embodiment of the first aspect, the fluoride salt of an amino acid ester is selected from the group consisting of Alanine-O-ethylester-ammoniumfluoride, Tyrosine-O-ethylester-ammoniumfluoride, Tryptophan-O-ethylester-N,N'-ammoniumdifluoride, and Lysine-O-ethylester-E-ammoniumdifluoride.

In one embodiment of the first aspect, the fluoride salt of an amino acid or the fluoride salt of an amino acid ester is less harmful to osteoblasts than olaflur. In preferred embodiments, the fluoride salt of an amino acid or the fluoride salt of an amino acid ester does not cause cell lysis when contacted with osteoblasts. Assays for determining the effect of the fluoride salts of the invention or of reference compounds are described in the Example section below.

In a second aspect the present invention is directed to the fluoride salt of an amino acid or the fluoride salt of an amino acid ester according to the first aspect for use in medicine.

In a third aspect the present invention is directed to the fluoride salt of an amino acid or the fluoride salt of an amino acid ester according to the first aspect for use in the prevention or treatment of caries.

In an alternative wording, the third aspect of the present invention is directed to the use of the fluoride salt of an amino acid or the fluoride salt of an amino acid ester according to the first aspect in the manufacture of a pharmaceutical composition for the prevention or treatment of caries.

In another alternative wording, the third aspect of the present invention is directed to a method for the prevention or treatment of caries in a subject in need thereof, the method comprising the step of:
applying the fluoride salt of an amino acid or the fluoride salt of an amino acid ester according to the first aspect to the teeth of the subject.

In one embodiment of the third aspect, the fluoride salt of an amino acid or the fluoride salt of an amino acid ester is to be administered orally. This means that the fluoride salt of an amino acid or the fluoride salt of an amino acid ester is to be applied to the oral cavity of the subject (e.g. when the fluoride salt is present in a mouth rinse). In preferred embodiments, the fluoride salt of the invention is to be directly applied onto the teeth of the subject (e.g. when the fluoride salt is present in a toothpaste or in a gel).

In some embodiments of the third aspect, the aspect of prevention of caries means prevention of the onset of caries.

In a fourth aspect the present invention is directed to a use of the fluoride salt of an amino acid or the fluoride salt of an amino acid ester according to the first aspect for mouth hygiene and/or dental care, wherein the use is non-therapeutic.

In a fifth aspect, the present invention is directed to a composition for mouth hygiene and/or dental care comprising the fluoride salt of an amino acid or the fluoride salt of an amino acid ester according to the first aspect, and optionally further comprising one or more of the following components:
a preservative (e.g. polyhexanide, taurolidine, benzalkonium chloride, chlorhexidine, octenidine and physiologically compatible salts thereof, preferably polyhexanide), a gelling agent (e.g. hydroxyethyl cellulose, hydroxymethyl cellulose, hydroxypropyl guar, methyl cellulose, ethyl cellulose, carbomers, alginates, gelatin, and poloxomer, preferably carbopol 940), a flavouring agent (e.g. *Mentha piperita* oil, Japanese peppermint oil, Eucalyptus oil, thyme oil, preferably *Mentha piperita* oil), a pH balancing agent (e.g. sodium bicarbonate or triethanolamine, preferably triethanolamine), a humectant (e.g. glycerol, sorbitol, or glycols, preferably glycerol), a bleaching agent (e.g. hydrogen peroxide, urea peroxide, or carbamide peroxide; preferably urea peroxide or carbamide peroxide), or a polishing agent (e.g. silica or mica, preferably mica).

In one embodiment of the fifth aspect, the composition is a dental bleaching product.

In one embodiment of the fifth aspect, the bleaching agent is urea peroxide. In a further embodiment of the fifth aspect, the urea peroxide is present in the composition in a concentration of up to 3% (w/v) and the composition is intended for non-therapeutic use. In another embodiment of the fifth aspect, the urea peroxide is present in the composition in a concentration of more than 3% (w/v) *[e.g. in a concentration of greater than 3% (w*/*v) and up to 30% (w*/*v); preferably in a concentration between 5% (w*/*v) and 25% (w*/*v); more preferably in a concentration between 10% (w*/*v) and 20% (w*/*v); even more preferably in a concentration between 12% (w*/*v) and 18% (w*/*v); and yet even more preferably n a concentration of about 15% (w*/*v)]* and the composition is intended for therapeutic use. In those embodiments, in which the composition is intended for therapeutic use, the composition is to be applied by a physician, a dentist or a dental assistant; preferably by a dentist or a dental assistant.

In one embodiment of the fifth aspect, the composition is a toothpaste, a tooth gel, a foam or a mouthwash solution.

In one embodiment of the fifth aspect, the fluoride salt of an amino acid or the fluoride salt of an amino acid ester is present in the composition in a concentration in the range between about 500 ppm and about 1500 ppm, preferably in a concentration in the range between about 750 ppm and about 1500 ppm, more preferably in a concentration in the range between about 1000 ppm and about 1500 ppm, even more preferably in a concentration in the range between about 1200 ppm and about 1400 ppm, and most preferably in a concentration of about 1400 ppm.

The following examples are merely illustrative of the present invention and should not be construed to limit the scope of the invention as indicated by the appended claims in any way.

### EXAMPLES

### Materials and Methods

Alanine-O-ethylester-ammoniumfluoride, Tyrosine-O-ethylester-ammoniumfluoride, Histidine-ammonium-difluoride, Tryptophan-N-ammonium-mono-hydrofluoride, Tryptophan-O-ethylester-N,N'-ammoniumdifluoride, Lysine-ε-ammonium-fluoride, Lysine-O-ethylester-E-ammoniumfluoride and Decalysine-ε-ammoniumdecafluoride, Dectaflur, Olaflur and MG-63 osteoblast cells were obtained from ELLY SWISS AG, CH-4422 Arisdorf, Switzerland.

### Synthesis of O-ethyl-L-alpha-amino acids

The O-ethyl-L-alpha-amino acids were prepared by ELLY SWISS AG. ELLY SWISS AG used *N*-protected t-BOC modified L-alpha-amino acids from Sigma-Aldrich which were converted into the corresponding t-BOC deprotected O-ethyl-L-alpha-amino-acids as described by G.SKORNA & I.UGI, Chemlnform Abstract: MILD ESTERIFICATION OF N-PROTECTED AMINO ACIDS VIA FOUR-COMPONENT CONDENSATION (4CC), Chemischer Informationsdienst, 10.1002/chin.197917353, 10, 17, (2016) [first published in February 1979 as G.Skorna & I.Ugi "Schonende Veresterung von N-terminal geschützten Aminosäuren mittels der Vier-Komponenten-Kondensation (4 CC)" Chem Ber 112(2):776-777 (1979)].

### Synthesis of the amino acid fluoride salts

To 100 ml of a 1 molar aqueous solution of the O-ethyl-L-alpha-amino-acids were added: **either** 50 ml of a 2 molar aqueous hydrofluoride solution for the neutral O-ethyl-L-alpha-amino-acids **or** 100 ml of a 2 molar aqueous hydrofluoride solution for all basic O-ethyl-L-alpha-amino-acids. The reactions were carried out below 25°C in a plastic (PE) vessel. The resulting pHs were between 6.7 and 7.4.

Synthesis of fluoride salts of basic amino acids were carried out in an analogous but simpler manner: the basic amino acid was directly reacted with an equimolar amount of an aqueous hydrofluoride solution.

### Assay:

The osteoblast cell-line used in this investigation was MG-63 (ATTC, human osteosarcoma cells) originating from a tumor of osteoblast tissue (Osteosarcoma) of a 14 year old male caucasian. The cells in tissue culture show a polygonal morphology with long dendrites. They show a similar sensitivity as primary human osteoblasts and are therefore relevant in cell research projects.

The cells were grown in tissue culture, 5 mg/ml Poly-HEMA treated, flasks with Dulbecco's Modified Eagle medium (DMEM) complemented with 10% fetal bovine serum (FBS) and 1% Gentamycin at 37°C and 5% CO₂.

Two days after inoculation, first cells became adherent developing dendrite structures. At this time cell culture media were supplemented with various fluoride compounds.

4 days after adding the fluorides, the cells were confluent on the bottom of the tissue culture vessel (control). Time to confluence was measured as a parameter of inhibition of cellular growth in the presence of the fluoride compounds relating to concentrations of 1400 ppm fluorides in the cell growth media and compared to the absence of fluorides (control).

### Results and discussion:

**Table 1: Time to confluence of osteoblasts in 5x10cm Tissue culture flasks**

| | Compounds (1400ppm) | Time to confluence (days) |
|---|---|---|
| | None (control) | 4-5 |
| Amino acid fluoride salts | Alanine-O-ethylester-ammoniumfluoride | 5-6 |
| | Tyrosine-O-ethylester-ammoniumfluoride | 5-6 |
| | Histidine-ammonium-difluoride | 7-8 |
| | Tryptophan-N-ammonium-mono-hydrofluoride | 5-6 |
| | Tryptophan-O-ethylester-N,N'-ammoniumdifluoride | 8-9 |
| | Lysine-ε-ammonium-fluoride | 4-5 |
| | Lysine-O-ethylester-ε-ammoniumdifluoride | 5-6 |
| | Decalysine-ε-ammonium-decafluoride | 8-9 |
| Amine fluorides of the prior art | Dectaflur | cell lysis |
| | Olaflur | cell lysis |
| Detergent | Sodium laurylsulfate (SDS) | cell lysis |
| Inorganic fluoride salt | Potassium fluoride | 6-7 |

Amino acid-fluorides have very low toxicity at commonly used 1400ppm fluoride concentration in dental hygiene products like toothpastes, gels and mouth rinses. Amino acid-ammonium-monofluorides and sodium fluoride show a 20% inhibition of cell growth except Lysine-ε-ammonium-fluoride with no inhibition at all. Amino acid-ammonium-difluorides and Decalysine-ε-ammonium-decafluoride show an growth inhibition of 40-50%.

Fluoride salts of N-alkylamines: Dectafluor and Olaflur and sodium laurylsulfate (SDS) were lysing (dissolving the membrane) osteoblast cells in culture. It was surprisingly found also in comparison with all L-amino acids in mammals that Lysine-ε-ammonium fluoride had no influence of growth on osteoblasts. This is an ideal compound for dental-hygiene products against caries and in tooth whitening formulations with maintained cellular activity.

These results are surprisingly positive for amino acid fluorides with little to no influence on osteoblast growth rates, i.e. low cell toxicity. Two selected formulations are presented in the examples, which can be applied to most of the existing commercial products (toothpastes, gels, mouth rinses).

### Examples for Formulations:

**Table 2: An anti-caries-tooth gel product**

| Ingredient | Composition in % (i.e. g/100 ml) |
|---|---|
| Lysine-ε-ammonium fluoride (anticaries agent) | 1.1 |
| Carbopol 940 (gelling agent) | 3.2 |
| Mentha piperita oil (flavoring agent) | 0.6 |
| Triethanolamine (pH balancing agent) | 0.34 |
| Polyhexanide (preservative) | 0.01 |
| Glycerol (humectant) | ad 100 ml |

**Table 3: Anti-caries and tooth whitening combination product**

| Ingredient | Composition in % (i.e. g/100 ml) |
|---|---|
| Lysine-ε-ammonium fluoride (anticaries agent) | 0.55-1.1 |
| Polyhexanide (preservative) | 0.0005-0.005 |
| Carbamide peroxide (bleaching agent, disinfectant) | 0.1-30 |
| Carbopol 940 (gelling agent) | 0.3-4.5 |
| Mentha piperita oil (flavoring agent) | 0.5-1.5 |
| Mica (polishing agent) | 0.0-1.0 |
| Glycerol (humectant) | ad 100 ml |

## Claims

1. A fluoride salt of an amino acid or a fluoride salt of an amino acid ester.

2. The fluoride salt of an amino acid or the fluoride salt of an amino acid ester of claim 1,
wherein the fluoride salt of an amino acid is selected from the group consisting of:
Histidine-ammonium-difluoride, Tryptophan-N-ammonium-mono-hydrofluoride, Lysine-ε-ammonium fluoride; decalysine-ε-ammonium-decafluoride.

3. The fluoride salt of an amino acid or the fluoride salt of an amino acid ester of claim 1,
wherein the fluoride salt of an amino acid ester is selected from the group consisting of Alanine-O-ethylester-ammoniumfluoride, Tyrosine-O-ethylester-ammoniumfluoride, Tryptophan-O-ethylester-N,N'-ammoniumdifluoride, and Lysine-O-ethylester-E-ammoniumdifluoride.

4. The fluoride salt of an amino acid or the fluoride salt of an amino acid ester of any one of claims 1 to 3 for use in medicine.

5. The fluoride salt of an amino acid or the fluoride salt of an amino acid ester of any one of claims 1 to 3 for use in the prevention or treatment of caries.

6. Use of the fluoride salt of an amino acid or the fluoride salt of an amino acid ester of any one of claims 1 to 3 for mouth hygiene and/or dental care, wherein the use is non-therapeutic.

7. A composition for mouth hygiene and/or dental care comprising the fluoride salt of an amino acid or the fluoride salt of an amino acid ester of any one of claims 1 to 3, and optionally further comprising one or more of the following components:
a preservative, a gelling agent, a flavouring agent, a pH balancing agent, a humectant, a bleaching agent, or a polishing agent.

8. The composition according to claim 7, wherein the composition is a dental bleaching product.

9. The composition according to claim 7 or 8, wherein the bleaching agent is urea peroxide.

10. The composition according to claim 9, wherein the urea peroxide is present in the composition in a concentration of up to 3% (w/v) and the composition is intended for non-therapeutic use.

11. The composition according to claim 9, wherein the urea peroxide is present in the composition in a concentration of more than 3% (w/v) and the composition is intended for therapeutic use.

12. The composition according to any one of claims 7 to 11, wherein the composition is a toothpaste, a tooth gel, a foam or a mouthwash solution.

13. The composition according to any one of claims 7 to 12, wherein the fluoride salt of an amino acid or the fluoride salt of an amino acid ester is present in a concentration in the range between about 500 ppm and about 1500 ppm, preferably in a concentration of about 1400 ppm.
